(19) Europäisches Patentamt
European Patent Office
Office européen des brevets

(11) Publication number : **0 500 254 A1**

(12) **EUROPEAN PATENT APPLICATION**

(21) Application number : **92301145.6**

(51) Int. Cl.$^5$ : **C07H 19/10,** C07H 19/20

(22) Date of filing : **12.02.92**

(30) Priority : **13.02.91 JP 40469/91**

(43) Date of publication of application : **26.08.92 Bulletin 92/35**

(84) Designated Contracting States : **CH DE FR GB LI NL**

(71) Applicant : **TSUMURA & CO.
4-10, Nihonbashi 3-chome
Chuou-ku, Tokyo 103 (JP)**

(72) Inventor : **Yamagami, Hidetomi
c/o Tsumura & Co., 3586 Yoshiwara
Ami-machi, Inashiki-gun, Ibaraki 300-11 (JP)**
Inventor : **Mori, Yoshikazu
c/o Tsumura & Co., 3586 Yoshiwara
Ami-machi, Inashiki-gun, Ibaraki 300-11 (JP)**

(74) Representative : **Stuart, Ian Alexander et al
MEWBURN ELLIS 2 Cursitor Street
London EC4A 1BQ (GB)**

(54) **Process for the synthesis of nucleoside-5'-alkylphosphonates.**

(57) A nucleoside-5′-alkylphosphonate is synthesized by reacting a nucleoside, optionally having protected hydroxyl groups at the 2′- and 3′-positions, with an alkylphosphonic halide, or reacting a nucleoside in which the hydroxyl groups at the 2′- and 3′-positions are protected with an alkylphosphonic acid or alkylphosphonic halide in the presence of a condensation agent.

EP 0 500 254 A1

Jouve, 18, rue Saint-Denis, 75001 PARIS

The present invention relates to a process for the synthesis of nucleoside-5′-alkylsulphonates useful as medicines such as an anti-tumor agent, for easy and great scale production thereof.

For reducing manufacturing expenses of pharmaceutical preparations, it is desirable to establish a more efficient synthesizing process therefore, and various studies have now been pursued aiming at the establishment of such a synthesizing process.

As the process for the synthesis of nucleoside-5′-alkylphosphonates, there have hitherto been known processes in which DCC (dicyclohexyl carbodiimide) is used as a condensation agent for alkylphosphonating the 5′-hydroxyl group of a nucleoside [Japanese Unexamined Patent Publication (Kokai) No. 1-224393, and Wojciech Rode et al., Biochemical Pharmacology, 36, 2, 203-210 (1987)].

In addition, as synthesizing processes similar to the above-described process, there have been known processes for the synthesis of nucleoside-5′-alkylphosphonates described in Kan Agarwal et al., Nucleic Acids Research, 6, 9, 3009-3024 (1979) and Paul S. Miller et al., Nucleic Acids Research, 11, 15, 5189-5204 (1983).

In the conventional synthesizing processes using DDC, however, a reaction time as long as about 96 to 120 hours is required, and complicated procedures are necessary for separating the produced nucleoside-5′-alkylphosphonates from the decomposition products thereof, or there are other problematic points, so that industrial large scale production of nucleoside-5′-alkylphosphonates has been difficult.

The present inventors continued intensive studies for solving the above-mentioned problem. As a result, there has been found a process whose preferred embodiments make possible the synthesis of nucleoside-5′-alkylphosphonates industrially easily and on an extensive scale in a short time.

In addition, the present inventors have found that when a condensation agent selected from the group consisting of the agents represented by the formulae (I), (II), (III) and (IV) as shown below is caused to act in reacting a nucleoside, in which the hydroxyl groups at the 2′- and 3′-positions are protected, with an alkylphosphonic acid or an alkylphosphonic acid halide, an improvement of the yield and simplification of purification of the product may be achieved, and thus accomplished the present invention.

Thus, the present invention provides a process for the synthesis of a nucleoside-5′-alkylphosphonate comprising reacting a nucleoside, optionally having protected hydroxyl groups at the 2′- and 3′- postions, with an alkylphosphonic halide

The present invention also provides a process for the synthesis of a nucleoside-5′-alkylphosphonate comprising reacting a nucleoside in which the hydroxyl groups at the 2′- and 3′-positions are protected with an alkylphosphonic acid or alkylphosphonic acid halide in the presence of a condensation agent selected from the group consisting of the agents represented by the following formulae (I), (II), (III) and (IV):

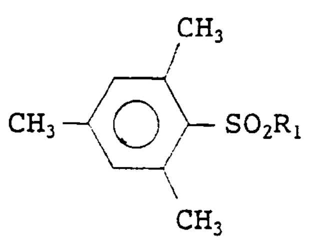

(I)

wherein $R_1$ stands for a chlorine atom or a group

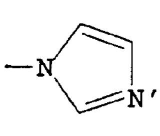 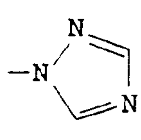 or 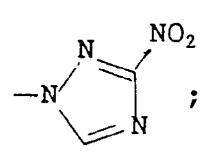 ;

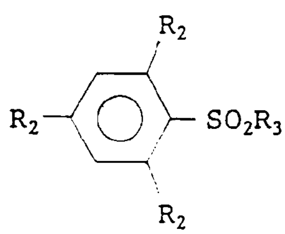

(II)

wherein $R_2$ stands for an isopropyl group, and $R_3$ stands for a chlorine atom or a group

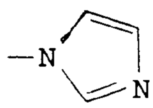 , 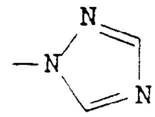 or 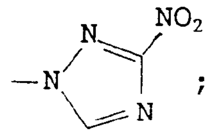 ;

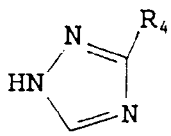

(III)

wherein $R_4$ stands for a hydrogen atom or a nitro group; and

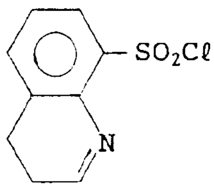

(IV)

DESCRIPTION OF THE PREFERRED EMBODIMENTS

The first process will now be explained below.

As the nucleoside, there may be mentioned, e.g., thymidine, cytidine, adenosine, guanosine, uridine, 5-fluorouridine, inosine, deoxycytidine, deoxyadenosine, deoxyguanosine, deoxyuridine, 5-fluorodeoxyuridine and the like, and a commercially available product may be used as the nucleoside. In addition, as the nucleoside, in which the hydroxyl groups at the 2′- and 3′-positions are protected, there may preferably be used a product obtained by reacting acetone or triethyl orthoformate and cyclopentanone with the above-described nucleoside in the presence of an acid catalyst such as p-toluenesulfonic acid. Alternatively, the hydroxyl groups may be protected by an acyl group such as acetyl, a silyl group such as trimethylsilyl, a benzyl group such as p-chlorobenzyl, a benzoyl such as benzoyl or a pyranyl group such as tetrahydropyranyl.

Any solvent may be used so long as it is an aprotic organic solvent with sufficient solvent power, which does not prevent the progress of the reaction. Specific examples of the solvent include, e.g., trimethylphosphate, triethylphosphate, dimethylformamide (DMF), dimethyl sulfoxide (DMSO), ethyl acetate, tetrahydrofuran (THF), dioxane and pyridine. They may be used singly or as a solvent mixture thereof.

The alkyl group of the used alkylphosphonic acid halide may preferably have 1 to 20 carbon atoms, and as the halogen of the halide, there may be mentioned chlorine, bromine and iodine. As the alkylphosphonic halide, there may be mentioned, e.g., methylphosphonic dichloride, methylphosphonic dibromide, methylphosphonic diiodide, ethylphosphonic dichloride, ethylphosphonic dibromide, ethylphosphonic diodide, propylphosphonic dichloride, propylphosphonic bromide, propylphosphonic dibromide and propylphosphonic diiodide.

The reaction may be carried out generally within the temperature range between -20 and 100°C, and especially preferably, it is reacted within the temperature range between 0°C and room temperature. Although the reaction time varies depending on the reaction substrate, the solvent and organic bases as described hereafter, it is generally within the range between about 2 and 48 hours. Nucleoside-5′-alkylphosphonates can be purified by combining conventional operations selected from extraction, ion exchange high performance liquid chromatography, reversed phase high performance liquid chromatography, ion exchange chromatography, recrystallization and the like.

In the above-mentioned reaction, in order to activate the reagent and neutralize the produced acid as occasion demands, there may be used, as a reaction auxiliary agent, an organic base such as tributylamine, pyridine, ammonia, dimethylaniline, triethylamine or γ-dimethylaminopyridine.

The process for the synthesis of a nucleoside used as the starting material, in which the hydroxyl groups at the 2′- and 3′-positions are protected, by giving an embodiment will be explained in the following.

Embodiment

400 mg of 5-fluorouridine (produced by Sigma Corporation) and 40 mg of p-toluenesulfonic acid were dissolved in 20 mℓ of anhydrous acetone, and the mixture was stirred for 3 hours at room temperature. Sodium hydrogencarbonate was added to the obtained reaction liquid under ice cooling and the mixture was allowed to stand overnight. An excessive amount of acetone was added to the mixture, and the salt thus separated out was separated by filtration, following which the obtained residue was washed with acetone, and the washing liquid and filtrate were mixed, and concentrated under reduced pressure, so that 460 mg of 2′, 3′-isopropylidene-5-fluorouridine was obtained, which was the chloroform soluble fraction of the obtained residue.

Next, the second process will be explained.

The nucleoside and the alkylphosphonic halide are identical with the above-described, respectively.

The alkylphosphonic acid to be used may be one containing 1 to 20 carbon atoms, and the specific examples thereof include, e.g., methylphosphonic acid, ethylphosphonic acid, propylphosphonic acid, butylphosphonic acid, pentylphosphonic acid, and hexylphosphonic acid.

As the solvent to be used, any may be used, so long as it is an aprotic organic solvent which has sufficient solvent power and does not prevent the progress of the reaction. The specific examples thereof include, e.g., dimethylformamide (DMF), dimethyl sulfoxide (DMSO), ethyl acetate, tetrahydrofuran (THF), dioxane and pyridine, which may be used singly or as a solvent mixture of two or more of them.

The reaction may be carried out generally at a temperature ranging from -20°C to 100°C, and is preferably, carried out within the temperature range between 0°C and room temperature. Although the reaction time varies depending on the reaction substrate, solvent and organic base, it is generally within the range between about 2.5 and 48 hours.

Nucleoside-5′-alkylphosphonates obtained by the foregoing procedure can be purified by combining the selected conventional operations such as extraction, ion exchange high performance liquid chromatography, reverse phase high performance liquid chromatography, ion exchange chromatography, recrystallization and the like.

In the above-mentioned reaction, in order to activate the reagent and neutralize the produced acid as occasion demands, there may be used, as a reaction auxiliary agent, an organic base such as tributylamine, ammonia, dimethylaniline, triethylamine or γ-dimethylaminopyridine.

In the second process of the present invention, the condensation agents of the formulae (I), (II), (III) and (IV) may be considered to accelerate the condensation of the alkylphosphonic acid or its halide with the nucleoside by activating the hydroxyl groups of the phosphonic acid moiety and at the 5′-position of the nucleoside.

In addition, the nucleoside-5′-alkylsulphonates synthesized according to the present invention may also be suitably obtained as bases, as occasion demands.

Nucleoside-5′-alkylsulphonates can be converted into the bases thereof either by reacting them with sodium perchlorate or the like in a solvent such as acetone etc., or by converting them into free acids using a cation exchange resin and then neutralizing the free acids with an alkali such as sodium hydroxide, sodium carbonate, calcium dihydroxide, calcium hydroxide etc.

Next, the present invention will be explained with reference to working examples for the synthesis of nucleoside alkylphosphonates, but it is to be noted that the present invention is not limited to these working examples.

Example 1

In 200 mℓ of a solution of trimethylphosphate, in which 52.4 g of commercially available 5-fluorouridine had been dissolved, there was added dropwise under ice cooling 100 ml of trimethylphosphate, in which 79.6 g of methylphosphonic dichloride had been dissolved. Further, 47.4 mℓ of tributylamine were added dropwise to the thus obtained solution, and the obtained solution mixture was stirred for 10 hours, whereafter ice water was added to the solution and stirred for a while, following which sodium hydrogencarbonate was added to neutralize the solution. Having been washed with dichloromethane, the solution was concentrated under reduced pressure. The salt deposited by suspending the residue in 600 mℓ of anhydrous methanol was separated by filtration, and added dropwise to 2ℓ of acetone under stirring. The separated white powder precipitate was subjected to centrifugal separation, and then subjected to anion exchange high performance liquid chromatography, following which the fraction eluted in a 0.1 M aqueous solution of triethylamine bicarbonate was collected and concentrated, so that 42.6 g of 5-fluorouridine-5′-methylphosphonate triethylamine salt was obtained.

The thus obtained compound was dissolved in 300 mℓ of methanol, and added dropwise under stirring to 1.5 ℓ of acetone in which 85.2 g of sodium perchlorate had been dissolved. The separated precipitate was subjected to centrifugal separation and then washed with acetone, so that 36 g (yield 49.5%) of white powder 5-fluorouridine-5′-methylphosphonate sodium salt were obtained.

mass spectrum m/e (%): 385 ($M^+$, +Na)

infrared absorption spectrum $\upsilon$ $KBr_{max}$ $cm^{-1}$:
3444, 1710, 1262, 1180, 1120, 1058
ultraviolet absorption spectrum $\lambda^{MeOH}_{max}$ nm (logε):
0.495 (8960)
proton nuclear magnetic resonance spectrum (δ ppm in $CDC\ell_3$):
1.33 (3H, d, J=16.4 Hz)
4.06 - 4.30 (5H, m)
5.92 - 5.97 (1H, m)
8.12 (1H, d, J=6.3 Hz)

Example 2

302 mg of 2′, 3′-O-isopropylidene-5-fluorouridine was dissolved in 10 mℓ of dioxane, and 398 mg of methylphosphonic dichloride was added to the obtained solution, whereafter 237 μℓ of tributylamine were added dropwise to the solution, and the obtained solution mixture was stirred for 10 hours. Ice water was added to the solution and the solution was neutralized with an aqueous solution of 5 % sodium hydrogencarbonate, whereafter the neutralized solution was washed with dichloromethane. After the water phase had been concentrated, methanol was added to the residue and the obtained solution was allowed to stand for a while, following which the deposited salt was separated by filtration, and then the filtrate was concentrated under a reduced pressure. 5 mℓ of 90% trifluoroacetic acid were added to the residue, and stirred for 4 hours at room temperature, whereafter the obtained homogeneous solution was concentrated under a reduced pressure. The residue was subjected to ion exchange chromatography (developing solvent: 0.1 to 0.2 M aqueous solution of triethylamine bicarbonate), and the eluted ultraviolet absorption fraction was collected and concentrated. The residue was dissolved in 3 mℓ of methanol and the obtained solution was added dropwise under stirring to 15 mℓ of acetone, in which 800 mg of sodium perchlorate had been dissolved. The separated precipitate was subjected to centrifugal separation and then washed with acetone, so that 246 mg (yield 68%) of white powder 5-fluorouridine-5′-methylphosphonate sodium salt were obtained.

mass spectrum m/e (%): 385 ($M^+$, +Na)

infrared absorption spectrum $\upsilon$ $KBr_{max}$ $cm^{-1}$:
3444, 1710, 1262, 1180, 1120, 1058
ultraviolet absorption spectrum $\lambda^{MeOH}_{max}$ nm (logε):
0.495 (8960)
proton nuclear magnetic resonance spectrum (δ ppm in $CDC\ell_3$):
1.33 (3H, d, J=16.4 Hz)
4.06 - 4.30 (5H, m)
5.92 - 5.97 (1H, m)
8.12 (1H, d, J=6.6 Hz)

Example 3

302 mg of 2′, 3′-O-isopropylidene-5-fluorouridine and 192 mg of methylphosphonic acid were twice azeotropically dehydrated with 5 mℓ of anhydrous pyridine, and then 2,4,6-triisopropylbenzene sulfonyl chloride dissolved in 3 mℓ of anhydrous pyridine was added to the dehydrated mixture, whereafter the solution was stirred for 4 hours at room temperature. The reaction liquid was concentrated under a reduced pressure, and partitioned with 20 mℓ of tributylamine and 30 mℓ of water, and the water phase was concentrated. 5 mℓ of 90% trifluoroacetic acid was added to the residue and stirred for 4 hours at room temperature, whereafter the obtained solution was concentrated under reduced pressure. The residue was subjected to ion exchange chromatography (developing solvent: 0.1 to 0.2 M aqueous solution of triethylamine bicarbonate), and the eluted ultraviolet absorption fraction was collected and concentrated. The residue was dissolved in 3 mℓ of methanol and the obtained solution was added dropwise under stirring to 15 mℓ of acetone, in which 800 mg of sodium perchlorate had been dissolved. The separated precipitate was subjected to centrifugal separation and washed with acetone, so that 245 mg of white powder 5-fluorouridine-5′-methylphosphonate sodium salt was obtained.

mass spectrum m/e (%): 385 ($M^+$, +Na)

infrared absorption spectrum $\upsilon$ $KBr_{max}$ $cm^{-1}$:

3444, 1710, 1262, 1180, 1120, 1058

ultraviolet absorption spectrum $\lambda^{MeOH}_{max}$ nm (logε):

0.495 (8960)

proton nuclear magnetic resonance spectrum (δ ppm in CDCℓ$_3$):

1.33 (3H, d, J=16.4 Hz)

4.06 - 4.30 (5H, m)

5.92 - 5.97 (1H, m)

8.12 (1H, d, J=6.6 Hz)

Example 4

302 mg of 2′, 3′-O-isopropylidene-5-fluorouridine and 192 mg of methylphosphonic acid were twice azeotropically dehydrated with 5 mℓ of anhydrous pyridine, and then 3-nitro-1H-1,2,4,-trazole dissolved in 3 mℓ of anhydrous pyridine was added to the dehydrated mixture, whereafter the solution was stirred for 28 hours at room temperature. The reaction liquid was concentrated under reduced pressure, and partitioned with 20 mℓ of methylene chloride, 2 mℓ of tributylamine and 30 mℓ of water, and the water phase was concentrated. 5 mℓ of 10% acetic acid was added to the residue and the obtained solution was stirred for 3 hours at a temperature of 100°C, whereafter the solution was concentrated under reduced pressure. The residue was subjected to ion exchange chromatography (developing solvent: 0.1 to 0.2 M aqueous solution of triethylamine bicarbonate), and the eluted ultraviolet absorption fraction was collected and concentrated. The residue was dissolved in 3 mℓ of methanol and the obtained solution was added dropwise under stirring to 15 mℓ of acetone, in which 800 mg of sodium perchlorate had been dissolved. The separated precipitate was subjected to centrifugal separation and washed with acetone, so that 264 mg of white powder 5-fluorouridine-5′-methylphosphonate sodium salt was obtained.

mass spectrum m/e (%): 385 ($M^+$, +Na)

infrared absorption spectrum $\upsilon\, KBr_{max}$ cm$^{-1}$:

3444, 1710, 1262, 1180, 1120, 1058

ultraviolet absorption spectrum $\lambda^{MeOH}_{max}$ nm (logε):

0.495 (8960)

proton nuclear magnetic resonance spectrum (δ ppm in CDCℓ$_3$):

1.33 (3H, d, J=16.4 Hz)

4.06 - 4.30 (5H, m)

5.92 - 5.97 (1H, m)

8.12 (1H, d, J=6.6 Hz)

Example 5

302 mg of 2′, 3′-O-isopropylidene-5-fluorouridine and 220 mg of ethylphosphonic acid were twice azeotropically dehydrated with 5 mℓ of anhydrous pyridine, and then 2,4,6-triisopropylbenzene sulfonyl chloride dissolved in 3 mℓ of anhydrous pyridine was added to the dehydrated mixture, whereafter the solution was stirred for 5 hours at room temperature. The reaction liquid was concentrated under reduced pressure, and partitioned with 20 mℓ of dichloromethane, 2 mℓ of tributylamine and 30 mℓ of water, and the water phase was concentrated. 5 mℓ of 90% trifluoroacetic acid was added to the residue and the obtained solution was stirred for 4 hours at room temperature, whereafter the solution was concentrated under reduced pressure. The residue was subjected to ion exchange chromatography (developing solvent: 0.1 to 0.2 M aqueous solution of triethylamine bicarbonate), and the eluted ultraviolet absorption fraction was collected and concentrated. The residue was dissolved in 3 mℓ of methanol and the obtained solution was added dropwise under stirring to 15 mℓ of acetone, in which 800 mg of sodium perchlorate had been dissolved. The separated precipitate was subjected to centrifugal separation and washed with acetone, so that a white powder substance was obtained. The thus obtained white powder substance was identified to be 5-fluorouridine-5′-ethylphosphonate sodium salt, for it exhibited the following physicochemical properties,

mass spectrum m/z (%): 377 ($M^+$, +Na)

infrared absorption spectrum $\upsilon\, KBr_{max}$ cm$^{-1}$:

3396, 1726, 1706, 1680, 1266, 1172

1086, 1042

proton nuclear magnetic resonance spectrum (δ ppm in $D_2O$):

0.88 - 1.78 (3H, m)

4.16 - 4.30 (3H, m)
5.83 - 5.96 (1H, m)
8.10 (1H, J = 6.6 Hz)

Example 6

302 mg of 2′, 3′-O-isopropylidene-5-fluorouridine and 220 mg of ethylphosphonic acid were twice azeotropically dehydrated with 5 mℓ of anhydrous pyridine, and then 3-nitro-1H-1,2,4,-triazole dissolved in 3 mℓ of anhydrous pyridine was added to the dehydrated mixture, whereafter the solution was stirred for 48 hours at room temperature. The reaction liquid was concentrated under a reduced pressure, and partitioned with 20 mℓ of dichloromethane, 2 mℓ of tributylamine and 30 mℓ of water, and the water phase was concentrated. 5 mℓ of 90% trifluoroacetic acid was added to the residue and the obtained solution was stirred for 4 hours at room temperature, whereafter the solution was concentrated under reduced pressure. The residue was subjected to ion exchange chromatography (developing solvent: 0.1 to 0.2 M aqueous solution of triethylamine bicarbonate), and the eluted ultraviolet absorption fraction was collected and concentrated. The residue was dissolved in 3 mℓ of methanol and the obtained solution was added dropwise under stirring to 15 mℓ of acetone, in which 800 mg of sodium perchlorate had been dissolved. The separated precipitate was subjected to centrifugal separation and washed with acetone, so that white powder 5-fluorouridine-5′-ethylphosphonate sodium salt was obtained.

mass spectrum m/z (%): 377 ($M^+$, +Na)

infrared absorption spectrum $\upsilon KBr_{max}$ $cm^{-1}$:
3396, 1726, 1706, 1680, 1266, 1172
1086, 1042

proton nuclear magnetic resonance spectrum (δ ppm in $D_2O$):
0.88 - 1.78 (3H, m)
4.16 - 4.30 (3H, m)
5.83 - 5.96 (1H, m)
8.10 (1H, J = 6.6 Hz)

Example 7

302 mg of 2′, 3′-O-isopropylidene-5-fluorouridine and 220 mg of ethylphosphonic acid were twice azeotropically dehydrated with 5 mℓ of anhydrous pyridine, and then 2, 4, 6-trimethylbenzene sulfonyl-3-nitrottriazolide dissolved in 3 mℓ of anhydrous pyridine was added to the dehydrated mixture, whereafter the solution was stirred for 3 hours at room temperature. The reaction liquid was concentrated under reduced pressure, and partitioned with 20 mℓ of dichloromethane, 2 mℓ of tributylamine and 30 mℓ of water, and the water phase was concentrated. 5 mℓ of 90% trifluoroacetic acid was added to the residue and the obtained solution was stirred for 4 hours at room temperature, whereafter the solution was concentrated under reduced pressure. The residue was subjected to ion exchange chromatography (developing solvent: 0.1 to 0.2 M aqueous solution of triethylamine bicarbonate), and the eluted ultraviolet absorption fraction was collected and concentrated. The residue was dissolved in 3 mℓ of methanol and the obtained solution was added dropwise under stirring to 15 mℓ of acetone, in which 800 mg of sodium perchlorate had been dissolved. The separated precipitate was subjected to centrifugal separation and washed with acetone, so that white powder 5-florouridine-5′-ethylphosphonate sodium salt was obtained.

mass spectrum m/z (%): 377 ($M^+$, +Na)

infrared absorption spectrum $\upsilon KBr_{max}$ $cm^{-1}$:
3396, 1726, 1706, 1680, 1266, 1172
1086, 1042

proton nuclear magnetic resonance spectrum (δ ppm in $D_2O$):
0.88 - 1.78 (3H, m)
4.16 - 4.30 (3H, m)
5.83 - 5.96 (1H, m)
8.10 (1H, J = 6.6 Hz)

Example 8

302 mg of 2′, 3′-O-isopropylidene-5-fluorouridine and 192 mg of methylphosphonic acid were twice azeot-

ropically dehydrated with 5 mℓ of anhydrous pyridine, and then 2, 4, 6-trimethylbenzene sulfonyl-3-nitrotriazolide dissolved in 3 mℓ of anhydrous pyridine was added to the dehydrated mixture, whereafter the solution was stirred for 2.5 hours at room temperature. The reaction liquid was concentrated under reduced pressure, and partitioned with 20 mℓ of dichloromethane, 2 mℓ of tributylamine and 30 mℓ of water, and the water phase was concentrated. 5 mℓ of 90% trifluoroacetic acid were added to the residue and the obtained solution was stirred for 4 hours at room temperature, whereafter the solution was concentrated under reduced pressure. The residue was subjected to ion exchange chromatography (developing solvent: 0.1 to 0.2 M aqueous solution of triethylamine bicarbonate), and the eluted ultraviolet absorption fraction was collected and concentrated. The residue was dissolved in 3 mℓ of methanol and the obtained solution was added dropwise under stirring to 15 mℓ of acetone, in which 800 mg of sodium perchlorate had been dissolved. The separated precipitate was subjected to centrifugal separation and washed with acetone, so that white powder 5-fluorouridine-5′-methylphosphonate sodium salt was obtained.

mass spectrum m/e (%): 385 ($M^+$, +Na)

infrared absorption spectrum $\upsilon KBr_{max}$ $cm^{-1}$:
3444, 1710, 1262, 1180, 1120, 1058
ultraviolet absorption spectrum $\lambda^{KBr}{}_{max}$ $cm^{-1}$:
0.495 (8960)
proton nuclear magnetic resonance spectrum (δ ppm in $CDC\ell_3$):
1.33 (3H, d, J = 16.4 Hz)
4.06 - 4.30 (5H, m)
5.92 - 5.97 (1H, m)
8.12 (1H, d, J = 6.6 Hz)

Example 9

After 2.8 g of propylphosphonic acid had been twice subjected to azeotropic distillation with 10 mℓ of pyridine, 3.0 g of 2′, 3′-O-isopropylidine-5-fluorouridine were added to the distillate, and the mixture was twice subjected to azeotropic distillation with 10 mℓ of pyridine, whereafter 7.6 g of 2,4,6-triisopropylbenzene sulfonyl chloride and 30 mℓ of pyridine were added to the distillate and the mixture was stirred for 4 hours at room temperature. 10 mℓ of water were added to the reaction liquid, and the solvent was distilled off, whereafter the residue was suspended in 50 mℓ of water, and the suspension was washed 3 times with 50 mℓ of dichloromethane, following which the water phase was concentrated. The residue was first dried under reduced pressure, and then purified by anion exchange chromatography (filler : DEAE, developing solvent : 0.05 to 0.5 M aqueous solution of triethylamine bicarbonate, linear gradient). The eluted ultraviolet absorption fraction was concentrated and the residue was dissolved in 100 mℓ of 10% acetic acid solution, whereafter the solution was refluxed for 2.5 hours at a temperature of 110°C. The solution was concentrated and dried under reduced pressure, whereafter the residue was subjected to cation exchange column and the eluted acid fraction was collected. The thus collected acid fraction was neutralized with 0.1 N aqueous solution of sodium hydroxide and concentrated under reduced pressure, so that 2.46 g of 5-fluorouridine-5′-propylphosphonate sodium salt was obtained.

mass spectrum m/e (%): 369.1 ($M^+$, +H)
391.1 ($M^+$, +Na)
proton nuclear magnetic resonance spectrum (δ ppm in $CD_3OD$):
1.03 - 1.07 (3H, m)
1.62 - 1.71 (2H, m)
1.77 - 1.86 (4H, m)
4.17 - 4.20 (3H, m)
4.22 - 4.32 (2H, m)
5.87 - 5.89 (1H, m)
7.92 - 7.94 (1H, d, J = 6.4 Hz)

Example 10

After 2.76 g of propylphosphonic acid had been twice subjected to azeotropic distillation with 10 mℓ of pyridine, 3.0 g of 2′, 3′-O-isopropylidine-5-fluorouridine were added to the distillate, and the mixture was twice subjected to azeotropic distillation with 10 mℓ of pyridine, whereafter 7.57 g of 2,4,6-triisopropylbenzene sulfonyl chloride and 30 mℓ of pyridine were added to the distillate and the mixture was stirred for 4 hours at room temperature. 10 mℓ of water were added to the reaction liquid, and the solvent was distilled off, whereafter the residue was suspended in 50 mℓ of water, and the suspension was washed 3 times with 50 mℓ of dichloromethane, following which the water phase was concentrated. The residue was first dried under reduced pressure, and then purified by anion exchange chromatography (filler : DEAE, developing solvent : 0.05 to 0.5 M aqueous solution of triethylamine bicarbonate, linear gradient). The eluted ultraviolet absorption fraction was collected and concentrated, following which the residue was dissolved in 100 mℓ of 10% acetic acid solution, and the solution was refluxed for 2.5 hours at a temperature of 110°C. The solution was concentrated and dried for 3 hours under reduced pressure, whereafter the residue was subjected to cation exchange column and the eluted acid fraction was collected. The thus collected acid fraction was neutralized with 0.1 N aqueous solution of sodium hydroxide and concentrated under a reduced pressure, so that 1.36 g of 5-fluorouridine-5′-butylphosphonate sodium salt was obtained.

mass spectrum m/e (%): 405.1 ($M^+$, +H)
428.1 ($M^+$, +Na)

proton nuclear magnetic resonance spectrum (δ ppm in $CD_3OD$):

0.89 - 0.93 (3H, m)
1.34 - 1.42 (2H, m)
1.55 - 1.66 (4H, m)
4.04 - 4.13 (3H, m)
4.18 - 4.25 (2H, m)
5.94 - 5.96 (1H, m)
7.96 - 7.98 (1H, d, J = 6.8 Hz)

Example 11

3.0 g of 2′, 3′-O-isopropylidine-5-fluorouridine were twice subjected to azeotropic distillation with 10 mℓ of pyridine, and 7.6 g of 2,4,6-triisopropylbenzene sulfonyl chloride and 30 mℓ of pyridine were added to the distillate and the mixture was stirred for 2 hours at room temperature. 4.1 g of hexylphosphonic dichloride was added to the reaction liquid, and the mixture was stirred for 6 hours at room temperature. 10 mℓ of water was added to the reaction liquid, and the solvent was distilled off, whereafter the residue was suspended in 50 mℓ of water, and the suspension was washed 3 times with 50 mℓ of dichloromethane, following which the water phase was concentrated. The residue was first dried under reduced pressure, and then purified by anion exchange chromatography (filler : DEAE, developing solvent : 0.05 to 0.5 M aqueous solution of triethylamine bicarbonate, linear gradient). The eluted ultraviolet absorption fraction was collected and concentrated, following which the residue was dissolved in 100 mℓ of 10% acetic acid solution, and the solution was refluxed for 2.5 hours at a temperature of 110°C. The solution was concentrated and dried for 3 hours under reduced pressure, whereafter the residue was subjected to cation exchange column and the eluted acid fraction was collected. The thus collected acid fraction was neutralized with 0.1 N aqueous solution of sodium hydroxide and concentrated under reduced pressure, so that 1.21 g of 5-fluorouridine-5′-hexylphosphonate sodium salt was obtained.

mass spectrum m/e (%): 433.1 ($M^+$, +H)
455.1 ($M^+$, +Na)

proton nuclear magnetic resonance spectrum (δ ppm in $CD_3OD$):

0.87 - 0.90 (3H, m)
1.29 - 1.39 (6H, m)
1.56 - 1.66 (4H, m)
4.05 - 4.16 (3H, m)
4.23 - 4.28 (2H, m)
5.94 - 5.95 (1H, m)
8.10 - 8.12 (1H, d, J = 6.4 Hz)

## Claims

1. A process for the synthesis of a nucleoside-5′-alkylphosphonate comprising reacting a nucleoside, optionally having protected hydroxyl groups at the 2′- and 3′- positions,with an alkylphosphonic halide.

2. A process according to claim 1, wherein the nucleoside is selected from thymidine, cytidine, adenosine, guanosine, uridine, 5-fluorouridine, inosine, deoxycytidine, deoxyadenosine, deoxyguanosine, deoxyuridine and 5-fluorodeoxyuridine.

3. A process according to claim 1, wherein the alkylphosphonic halide is selected from methylphosphonic dichloride, methylphosphonic dibromide, methylphosphonic diiodide, ethylphosphonic dichloride, ethylphosphonic dibromide, ethylphosphonic diiodide, propylphosphonic dichloride, propylphosphonic bromide, propylphosphonic dibromide and propylphosphonic diiodide.

4. A process according to any preceding claim wherein the reaction is carried out in an aprotic organic solvent selected from trimethylphosphate, triethylphosphate, dimethylformamide, dimethyl sulfoxide, ethyl acetate, tetrahydrofuran, dioxane and pyridine.

5. A process according to any preceding claim wherein an organic base selected from tributylamine, pyridine, ammonia, dimethylaniline, triethylamine and γ-dimethylaminopyridine is used as a reaction auxiliary agent.

6. A process according to any preceding claim wherein the reaction is carried out at a temperature of from -20 to 100°C for 2 to 48 hours.

7. A process for the synthesis of a nucleoside-5′-alkylphosphonate comprising reacting a nucleoside in which the hydroxyl groups at the 2′- and 3′-positions are protected with an alkylphosphonic acid or alkylphosphonic halide in the presence of a condensation agent selected from the group consisting of the agents represented by the following formulae (I), (II), (III) and (IV):

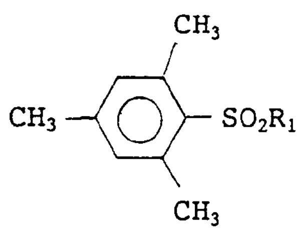

(I)

wherein $R_1$ stands for a chlorine atom or a group

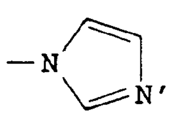 , 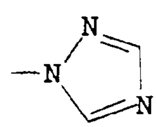 or 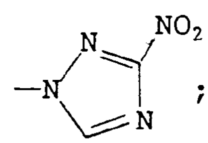 ;

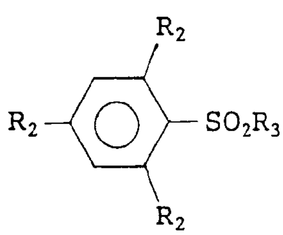

(II)

wherein $R_2$ stands for an isopropyl group, and $R_3$ stands for a chlorine atom or a group

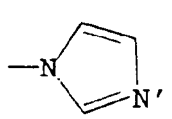 , 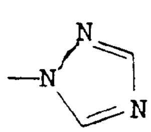 or 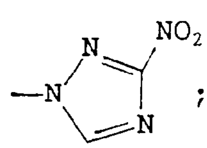 ;

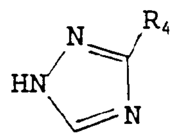

(III)

wherein $R_4$ stands for a hydrogen atom or a nitro group; and

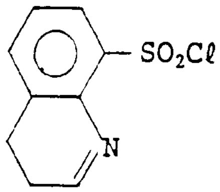

(IV)

**8.** A process according to claim 7, wherein the nucleoside is selected from thymidine, cytidine, adenosine, guanosine, uridine, 5-fluorouridine, inosine, deoxycytidine, deoxyadenosine, deoxyguanosine, deoxyuridine and 5-fluorodeoxyuridine.

**9.** A process according to claim 7 or 8 wherein the alkylphosphonic acid is selected from methylphosphonic acid, ethylphosphonic acid, propylphosphonic acid, butylphosphonic acid, pentylphosphonic acid, and hexylphosphonic acid.

**10.** A process according to claim 7 or 8 wherein the alkylphosphonic halide is selected from methylphosphonic dichloride, methylphosphonic dibromide, methylphosphonic diiodide, ethylphosphonic dichloride, ethylphosphonic dibromide, ethylphosphonic diiodide, propylphosphonic dichloride, propylphosphonic bromide, propylphosphonic dibromide and propylphosphonic diiodide.

**11.** A process according to any of claims 7-10 wherein the reaction is carried out in an aprotic organic solvent selected from dimethylformamide, dimethyl sulfoxide, ethyl acetate, tetrahydrofuran, dioxane and pyridine.

**12.** A process according to any of claims 7-11 wherein an organic base selected from tributylamine, ammonia, dimethylaniline, triethylamine and γ-dimethylaminopyridine is used as a reaction auxiliary agent.

**13.** A process according to any of claims 7-12 wherein the reaction is carried out at a temperature of from -20 to 100°C for 2.5 to 48 hours.

European Patent Office

# EUROPEAN SEARCH REPORT

Application Number

EP 92 30 1145

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | JOURNAL OF MEDICINAL CHEMISTRY.<br>vol. 32, no. 2, February 1989, WASHINGTON US<br>pages 367 - 374;<br>R.W.LAMBERT ET AL.: 'Synthesis and Antiviral Activity of Phosphonoacetic and Phosphonoformic Acid Esters of 5-Bromo-2'-Deoxyuridine and Related Pyrimidine Nucleosides and Acyclonucleosides'<br>* the whole document *<br>--- | 1-13 | C07H19/10<br>C07H19/20 |
| D,X | BIOCHEMICAL PHARMACOLOGY<br>vol. 36, no. 2, 1987,<br>pages 203 - 210;<br>W.RODE ET AL.: 'Studies on the Interaction with Thymidylate Synthase of Analogues of 2'-Deoxyuridine-5'-Phosphate and 5-Fluoro-2'-Deoxyuridine-5'-Phosphate with Modified Phosphate Groups.' | 1-6 | |
| Y | * the whole document *<br>--- | 1-13 | |
| Y | US-A-4 056 673 (E.P.HEIMER ET AL.)<br>* the whole document *<br>--- | 1-13 | TECHNICAL FIELDS SEARCHED (Int. Cl.5) |
| Y | EP-A-0 375 183 (SCHERING CORPORATION)<br>* page 12, line 14 - page 12, line 39 *<br>--- | 1-13 | C07H |
| Y | EP-A-0 097 376 (FARMITALIA CARLO ERBA S.R.L.)<br>* abstract *<br>* page 6, line 8 - line 12 *<br>--- | 1-13 | |
| P,X | WO-A-9 104 261 (FARMITALIA CARLO ERBA S.R.L.)<br>* the whole document *<br>----- | 1-13 | |

The present search report has been drawn up for all claims

| Place of search | Date of completion of the search | Examiner |
|---|---|---|
| THE HAGUE | 12 JUNE 1992 | SCOTT J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)